# FASCICULE DE BREVET EUROPEEN

(11) **EP 1 742 063 B1**
(45) Date de publication et mention de la délivrance du brevet: **08.09.2010**
(21) Numéro de dépôt: 05014751.1
(22) Date de dépôt: 07.07.2005
(51) Int. Cl.: G01N 33/86, G01N 33/49

(54) **Système de détermination différentielle du taux d'une enzyme protéolytique dans un fluide corporel**
System zur differenziellen Bestimmung der Menge eines proteolytischen Enzyms in einer Körperflüssigkeit
System for the differential determination of the amount of a proteolytic enzyme in a body fluid.

(43) Date de publication de la demande: 10.01.2007
(73) Titulaire: ASULAB S.A., 2074 Marin (CH)
(72) Inventeur: Frenkel, Erik Jan, 2009 Neuchatel (CH); Thuerlemann, Charles, 9322 Egnach (CH); Haeberli, André, 3073 Gümligen (CH)
(74) Mandataire: Couillard, Yann Luc Raymond

(56) Documents cités:
- EP-A- 0 471 986
- EP-A- 1 031 830
- US-A- 6 066 504
- US-B1- 6 495 336
- CHARLES BERNARD THÜRLEMANN: "Entwicklung eines Biosensor-Systems für ein Patienten-Selbstmanagement der Behandlung mit Vitamin K - Antagonisten" E-DISS UNI BASEL, [Online] 2005, XP002369808 Basel Extrait de l'Internet: URL:http://pages.unibas.ch/diss/2005/DissB _7319.pdf> [extrait le 2006-02-27]

## Description

### DOMAINE TECHNIQUE

L'invention cvnceme un système permettant de mettre en oeuvre un test pour déterminer de façon différentielle en temps réel l'évolution du taux d'une enzyme protéolytique dans un faible échantillon de fluide corporel, et en fonction de cette analyse différentielle dynamique de pouvoir prévenir la tendance d'un patient à développer une pathologie donnée.

L'invention sera plus particulièrement illustrée par le test de génération de thrombine, par abréviation TGT, plus connu en anglais par l'abréviation ETP (Endogenous Thrombine Potential), dans lequel la mesure en continu du taux de certains facteurs plasmatiques de coagulation permet de détecter des taux anormaux et d'anticiper par une traitement approprié un risque d'hémophilie, ou au contraire de thrombose.

L'invention cvnceme également un système de laboratoire, qui pourrait éventuellement être adapté pour effectuer les mesures au chevet du malade.

### ARRIERE PLAN TECHNOLOGIQUE

Connaître le temps de coagulation du sang, désigné par temps de prothrombine (par abréviation PT), c'est-à-dire l'aptitude de différentes enzymes protéolytiques, également désignées par "facteurs", à concourir à la formation d'un caillot ou au contraire à la prévenir, fait partie des examens de routine, voire d'examen journaliers dans de nombreuses situations pathologiques acquises, traumatiques, pré- ou post-opératoires. Il est par exemple nécessaire lors d'un traitement par anticoagulants dans le cas de maladies cardiaques de pouvoir ajuster avec précision la posologie d'un médicament anti-thrombotique, par exemple la warfarin ou l'héparine, afin d'éviter les risques hémorragiques en cas de surdosage, ou au contraire les risques de thrombose si la posologie d'anticoagulants est insuffisante.

Cette détermination du temps de prothrombine (PT) ou du temps de thromboplastine partiellement activée (APTT) a longtemps été effectuée en laboratoire par observation visuelle directe du temps nécessaire à la formation d'un caillot, puis avec l'aide d'appareils plus ou moins complexes et encombrants faisant le plus souvent appel à une détection optique, tels que décrits par exemple dans les brevets US 5 302 348 et US 5 154 082.

Selon des méthodes plus récentes le principe consiste à utiliser un substrat chimique incorporant au moins un réactif chimique dont un maillon terminal peut être coupé par une enzyme spécifique pour libérer un groupe (LG) dont on peut détecter la présence dans le milieu de mesure avec un signal représentatif de l'activité de l'enzyme.

Une telle méthode de détection correspond par exemple à celle décrite dans le brevet EP 0 679 193. Dans la méthode décrite, un capteur comporte un substrat chimique dont un maillon terminal peut être coupé par l'enzyme à analyser pour libérer un groupe (LG) dont la concentration représentative de l'activité de ladite enzyme dans le milieu peut être mesurée par des moyens optiques basés sur des modifications de colorimétrie, luminescence ou fluorescence. Lorsque le fluide corporel à analyser est du sang entier, il est nécessaire d'éliminer les érythrocytes, soit par centrifugation préalable de l'échantillon, soit en prévoyant au niveau du capteur un membrane formant barrière aux érythrocytes. Cette méthode a donc l'inconvénient de nécessiter un temps d'analyse relativement long, voire coüteux, pour l'élimination des érythrocytes.

L'inconvénient sus-indiqué peut être fortement réduit, voire éliminé, avec la méthode proposée dans le brevet EP 1 031 830, incorporé dans la présente demande par référence. La méthode, qui concerne la mesure du temps de coagulation du sang, repose également sur la détermination indirecte de l'activité d'une enzyme protéolytique au moyen d'un substrat chimique capable de libérer, sous l'action de ladite enzyme des groupes chargés (LG) qui vont modifier les propriétés électriques du milieu, le signal en résultant étant dans ce cas analysé par ampérométrie et corrélé à une valeur représentative, PT ou APTT du temps de coagulation. Avec cette méthode non colorimétrique, on peut se passer d'une préparation préalable pour avoir un plasma clair, et effectuer plus rapidement la détermination sur du sang entier.

Toutes les méthodes qui viennent brièvement d'être rappelées permettent seulement d'effectuer une détermination globale et ne permettent pas de connaître, parmi toutes les enzymes qui interviennent dans le phénomène de coagulation, l'enzyme responsable d'une anomalie de la coagulation, qu'il s'agisse d'hémophilie ou de thrombose.

Jusqu'à une époque récente, pour obtenir ce genre d'information, la méthode consistait à séparer un prélèvement de sang en plusieurs échantillons et à faire réagir différents anticorps pour repérer quelle enzyme était défectueuse. Cette méthode nécessitait un prélèvement de sang relativement important, demandait beaucoup de temps et ne pouvait être exécutée qu'en laboratoire.

Plus récemment la demande internationale WO 03/093831 décrit une méthode permettant de déterminer en temps réel l'évolution de l'activité de la thrombine dans un échantillon de sang, mais de préférence dans un échantillon de plasma, en faisant appel à une détermination fluorométrique, comparativement à une courbe de calibration. Cette méthode présente les mêmes inconvénients que ceux précédemment évoqués pour une détermination globale du temps de prothrombine, concernant en particulier le volume de l'échantillon relativement important (environ 160µl, dont 80µl pour la solution de calibration), et le temps de mesure assez long (environ 45 minutes).

Enfin, le document WO 00/033074 divulgue un appareil de mesure destiné à recevoir un à un dans une même borne plusieurs types de capteurs sous forme de bandelette et capables d'interagir chacun avec un composé particulier. Chaque capteur comporte un repère correspondant à son réactif qui rend possible sa détection par l'appareil de mesure une fois introduit dans sa borne. L'appareil de mesure n'a pas à être préparé pour une mesure puisqu'il reconnaît automatiquement quel type de capteur est introduit dans son unique borne ce qui lui permet d'appliquer le protocole idoine.

### RESUME DE L'INVENTION

La présente invention vise donc à pallier les inconvénients de l'art antérieur précité en procurant un test de détermination différentielle de l'évolution du taux d'une enzyme protéolytique au cours du temps et notamment pour effectuer sur un échantillon de sang complet ou de plasma de faible volume, et dans un temps relativement court, en quelque sorte un screening de l'activité des enzymes impliquées dans le phénomène de coagulation.

A cet effet l'invention a pour objet un système de détermination électrochimique de l'évolution de la concentration ou de l'activité d'au moins une enzyme protéolytique pour en détecter une éventuelle carence ou une activité anormale dans un faible échantillon d'un fluide corporel, tel que du plasma ou du sang entier.

Le système comprend un assortiment de capteurs électrochimiques, un appareil de mesure et un appareil de traitement d'un signal électrique.

Chaque capteur a la forme d'une languette de petites dimensions supportant au moins une électrode de référence et une électrode de travail sur laquelle est immobilisé un réactif spécifique d'un enzyme protéolytique donnée, dont la composition incorpore au moins un substrat chimique dont un maillon terminal peut être coupé par ladite enzyme pour libérer des groupes chargés (LG).

L'appareil de mesure comporte au moins une fente de connexion destinée à recevoir un capteur, et un circuit électronique alimenté par une source d'énergie permettant d'imposer entre les électrodes du capteur un courant électrique ayant une intensité ou une tension variable ou non, et de recevoir en retour un signal électrique représentatif de la libération des groupes chargés (LG).

Dans un mode de réalisation préféré l'appareil de mesure permet d'effectuer une détermination chrono-ampérométrique.

L'appareil électronique comporte un software permettant le traitement du signal émis par l'appareil de mesure pour afficher sur un écran de visualisation une indication représentative de la libération des groupes chargés (LG) en fonction du temps. Cette information peut être donnée sur l'écran sous forme alphanumérique, ou sous forme de courbes affichées de façon séquentielle ou en mosaïque.

### BREVE DESCRIPTION DES DESSINS

D'autres caractéristiques et avantages de la présente invention apparaîtront plus clairement dans la description ci-après d'un exemple de réalisation, donné à titre illustratif et non limitatif, en référence aux dessins annexés dans lesquels:
- la figure 1 représente en perspective un système de mesure selon l'invention;
- la figure 1A représente une variante de capteur qui ne fait pas partie de l'invention;
- la figure 2 est un schéma du processus conduisant à un signal exploitable par l'appareil de mesure, puis par le software de l'appareil électronique;
- la figure 3 comporte une courbe représentative d'un carence en facteur II;
- la figure 4 comporte une courbe représentative d'une activité anormale du facteur V Leiden;
- la figure 5 comporte une courbe représentative d'une carence en facteur VII, et
- la figure 6 comporte une courbe représentative d'une carence en protéine S.

### DESCRIPTION DETAILLEE DE L'INVENTION

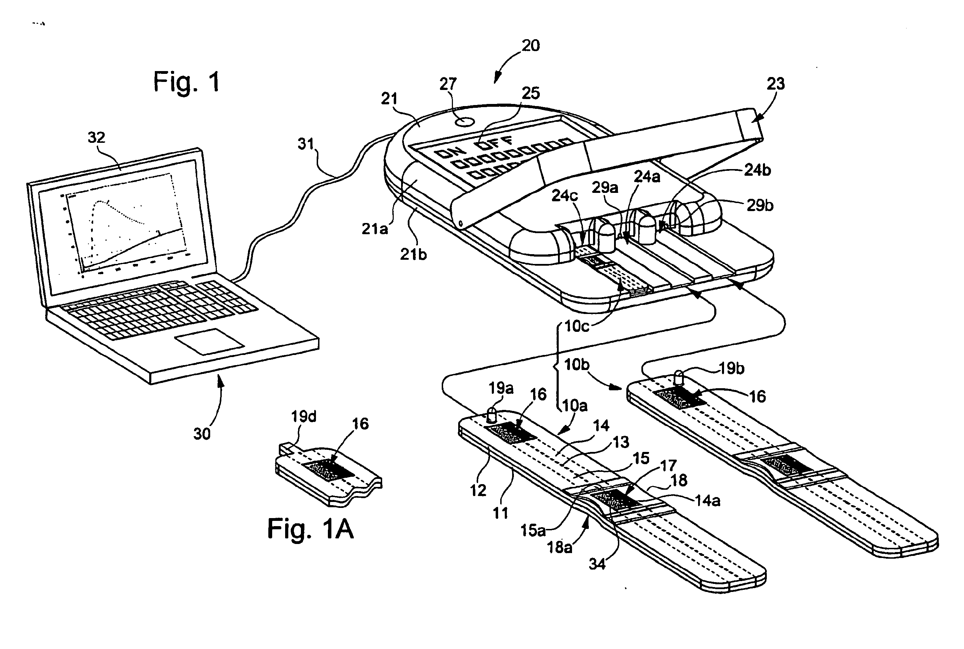

En se référant d'abord à la figure 1, on a représenté à titre d'exemple un système selon l'invention permettant d'effectuer un screening du sang d'un patient, éventuellement au chevet de celui-ci. Le système comprend un assortiment 10 de capteurs électrochimiques, un appareil de mesure 20 et un appareil électronique 30, ces trois éléments n'étant pas représentés à la même échelle.

Dans l'éxemple représenté, l'assortiment 10 comprend trois capteurs 10a, 10b et10c représentés par commodité à plus grande échelle. Chaque capteur à la forme d'une languettes d'environ 40mm de long et 8mm de large.

En se référant plus particulièrement au capteur 10a, destiné par exemple à détecter une déficience en prothrombine (également désigné par facteur Il), on voit qu'il comporte un support plastique mince 11, réalisé par exemple en PET, supportant sur toute sa longueur deux collecteurs de courant 14, 15 séparés par un petit espace 13 qui les isole électriquement.

Le support 11 et les collecteurs 14, 15 sont recouverts d'un revêtement isolant 12 dans lequel sont découpées, par exemple par estampage près de chaque extrémité, deux fenêtres 16 et 17 laissant apparaître des portions des collecteurs 14, 15. Une première fenêtre 16 permet de connecter électriquement le capteur 10a à l'appareil de mesure 20. La deuxième fenêtre 17 constitue la zone de mesure, les portions visibles des collecteurs formant respectivement l'électrode de travail 14a et l'électrode de référence 15a.

L'électrode de travail 14a est par exemple réalisée par laminage d'une mince bande de platine et l'électrode de référence 15a par laminage d'une mince bande d'argent, préalablement ou ultérieurement chlorurée. Il est également possible de prévoir une contre-électrode dans la zone de mesure. L'électrode de travail 14a est revêtue d'un réactif spécifique 34 décrit en détails plus loin.

Dans la modèle de capteur représenté, on voit que la fenêtre de mesure 17 est recouverte d'un capot transparent 18, formant un canal capillaire transversal 18a permettant d'amener l'échantillon de sang à analyser en contact avec les électrodes 14a et 15a.

On voit que l'extrémité du capteur comporte un repérage 19 spécifique d'un type de capteur de l'assortiment, permettant à l'appareil de mesure 20 de le "reconnaître". Pour le capteur 10a ce repérage est constitué par un bossage 19a situé dans l'axe du capteur. Pour le capteur 10b, le bossage 19b est déporté à droite et pour le capteur 10c, représenté en place dans l'appareil de mesure 20, le bossage 19c (non visible) est déporté à gauche. Il est également possible d'envisager d'autres types de repérage, qui ne fait cependant pas partie de l'invention, par exemple une petite extension 19d de l'extrémité du capteur, comme représenté à la figure 1A, ou au contraire une petite encoche (non représentée). L'intérêt de ces moyens de repérage apparaîtra plus clairement avec la description de l'appareil de mesure 20.

On observera également que l'assortiment 10 peut comporter un plus grand nombre de capteurs, incluant avantageusement un capteur de calibration

L'appareil de mesure 20 comprend un boîtier 21 construit par assemblage de deux coques en plastique moulé 21 a, 21b, la coque inférieure 21b s'étendant un peu au-delà de la coque supérieure 21 a. Ces deux coques délimitent un logement pour une source d'énergie et pour un circuit électronique (non représentés) du traitement des signaux émis par les groupes chargés (LG). Ce circuit électronique est une adaptation des circuits utilisés pour le dosage du glucose, par exemple par ampérométrie comme décrit dans le brevet US 5 378 628. Il n'en diffère que par un paramétrage différent du signal électrique représentatif de la libération des groupes LG par la thrombine, ou par d'autres protéines protéolytiques.

L'appareil de mesure 20 comporte également autant de fentes de connexion 24a. 24b, 24c que de capteurs 10a, 10b, 10c dans l'assortiment du système de mesure. Ces fentes de connexion sont réalisées entre et dans les coques 21 a et 21 b formant le boîtier 21, Dans le mode de réalisation représenté, la coque supérieure présente un encoche et la coque inférieure une rainure en creux permettant d'introduire et de retirer le capteur jetable après usage.

Selon la variante de réalisation représentée, l'appareil de mesure 20 comporte en outre un capot rabattable 23 qui permet d'isoler les capteurs 10a, 10b, 10c introduits dans l'appareil qui comporte alors un thermostat (non représenté) permettant de maintenir la zone de mesure à une température constante (par exemple 37°C). Comme on le verra par la suite, la température a une très grande influence sur la génération de thrombine. De façon alternative il serait possible de se passer d'une enceinte thermostatée en prévoyant une sonde (non représentée) mesurant la température ambiante et permettant de sélectionner une courbe de calibration parmi un ensemble de courbes, mis en mémoire dans l'appareil de mesure ou mieux dans l'appareil électronique, en fonction de différentes températures.

On remarquera aussi que chaque fente de connexion 24a, 24b et 24c comporte une encoche 29a, 29b et 29c (non visible) complémentaire de chaque bossage de repérage 19a, 19b et 19c, c'est-à-dire empêchant toute interversion de capteur et permettant de désigner en clair et sans erreur la courbe ou les informations qui s'afficheront sur l'écran de visualisation.

Avec le type de repérage représenté à la figure 1A, qui ne fait cependant pas partie de l'invention la "reconnaissance" d'un capteur déterminé peut être effectuée par voie électronique par l'appareil de mesure 20, de sorte que n'importe quelle fente de connexion peut recevoir n'importe quel capteur. Ce type de repérage augmente également le nombre de capteurs différents que peut comporter un assortiment 10. Par exemple, avec un nombre d'extension 19d compris entre zéro et 3 pouvant occuper trois positions différentes, on peut différencier huit capteurs.

On observera enfin que l'appareil de mesure 20 peut éventuellement comporter un affichage secondaire 25, pouvant servir d'écran de contrôle de bon fonctionnement, par exemple en affichant ON ou OFF selon que le bouton de commande 27 a été pressé ou non, ou en donnant une information visuelle de fin de mesure permettant de retirer les capteurs en toute sécurité. L'affichage secondaire peut également afficher, à titre d'information complémentaire, une valeur globale de mesure tel que PT ou APTT.

L'appareil de mesure 20 qui vient d'être décrit comporte trois fentes de connexion 24a, 24b et 24c, mais il est bien évident qu'il pourrait en comporter un plus grand nombre, pour permettre d'effectuer un plus grand nombre de déterminations simultanées.

L'appareil de mesure 20 est connectable par un cordon 31 à un appareil électronique 30 comportant un écran d'affichage 32. Dans l'exemple représenté, l'appareil électronique est un ordinateur portable dans lequel a été installé un software permettant de traiter les signaux reçus de l'appareil de mesure 20 pour afficher sur l'écran 32 des courbes ou des informations sur la détermination en cours. L'ordinateur permet également au praticien de mémoriser les informations qui lui sont utiles pour l'interprétation des courbes, et/ou qui lui permettent de suivre la pathologie d'un patient donné, ainsi que d'effectuer les taches ordinaires possibles avec un ordinateur.

Dans l'exemple de la figure 1, l'appareil de mesure 20 et l'appareil électronique 30 sont représentés par des éléments séparés, mais il est tout à fait concevable de les réunir dans une même unité fonctionnelle. Il est même possible de concevoir tout l'ensemble sous forme d'une mallette comportant un logement de rangement pour des assortiments 10 de capteurs.

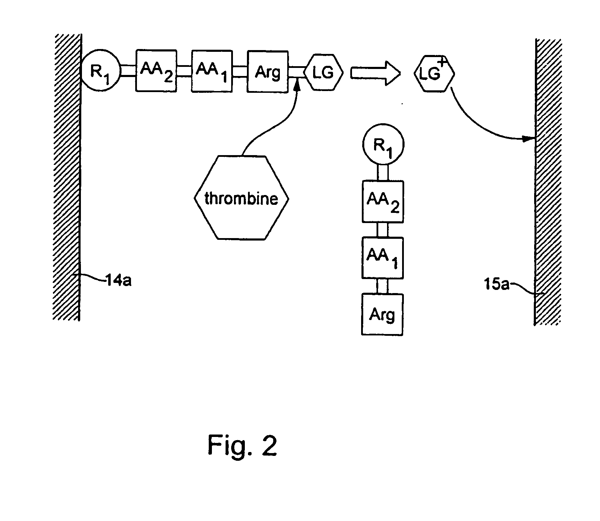

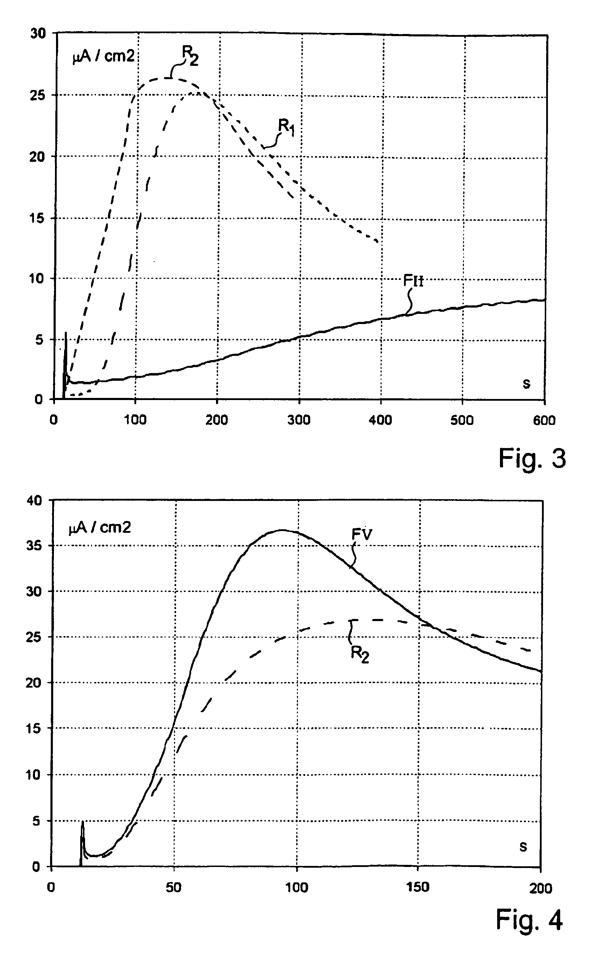

La figure 2 correspond à une représentation schématique de la réaction qui permet de générer un courant entre les électrodes 14a et 15a qui sont reliées par un circuit électronique de détection non représenté. Le substrat est représenté schématiquement par la formule R₁-AA₂-AA₁-Arg-LG dans laquelle AA₁ et AA₂ représentent des amino-acides tels que ceux décrits dans les brevets US 4 304 853 ou US 6 352 853, mais il est tout à fait possible d'utiliser d'autres peptides. Le groupe R₁ représente un groupe de liaison avec l'électrode de travail 14a permettant d'orienter l'oligopeptide et LG représente un groupe séparable ("leaving group") chargé, tel que l'un des groupes décrit dans le brevet US 6 352 853 précité. Dans la partie gauche du schéma, on voit que l'enzyme thrombine coupe sélectivement la liaison entre l'arginine et le "leaving group" LG. Dans la partie droite du schéma, on voit que le "leaving group" libéré peut migrer vers l'électrode 15a et engendrer un courant qui sera proportionnel au nombre de "leaving group" LG libérés et donc à la quantité de thrombine formée dans le milieu par unité de temps. En d'autres termes, la détermination de l'activité d'une enzyme protéolytique donnée repose sur une mesure chrono-ampérométrique permettant de tracer une courbe représentant la variation de l'intensité en µA/cm² en fonction du temps exprimé en secondes comme représenté dans les graphiques des figures 3 à 6. Une telle détermination chrono-ampérométrique permet également, au moyen d'un algorithme de calcul approprié, d'afficher les résultats de mesure, par exemple en valeur ETP (potentiel de thrombine endogène).

Dans la méthode concernant une détermination globale (PT ou APTT) la valeur retenue est, par exemple celle du point d'inflexion, mesuré environ dans les 15 secondes qui suivent le début de la réaction et cette valeur ne représente que 10% environ de la thrombine totale. Avec le test selon l'invention le temps d'enregistrement est nettement plus long, pouvant aller jusqu'à 45mm, de préférence entre 2 et 30 mn et en particulier entre 3 et 10mn. Cela permet de prendre en compte les paramètres importants pour le praticien concernant la "dynamique" de la génération de thrombine, comme cela est expliqué plus en détail en référence à la figure 3.

La figure 3 correspond à un graphique montrant une déficience en facteur Il, à savoir la prothrombine dans le plasma. Elle a été réalisée avec un capteur dont la surface de l'électrode de travail est de 0,054cm² en prenant comme substrat l'oligopoptide TOS-Gly-Pro-Arg-3chloro-4-hydroxyanilide, 2HCl.

Il est bien évidemment possible de faire d'autres choix, tant en ce qui concerne la nature du substrat, que la surface de l'électrode de travail.

L'enregistrement a été effectué avec du plasma normal à la température constante de 23,5°C pour la courbe de référence R₁ et pour la courbe de mesure FII. Dans chaque cas la quantité d'échantillon déposé, ou une solution de référence, est de 10 µl. La détermination a été effectuée sur une durée de 10 minutes.

Sur la figure 3 on a également représenté une deuxième courbe de référence R₂, avec du plasma normal, enregistrée à la température de 24,5°C, et qui montre clairement qu'une différence de seulement 1°C provoque un déplacement significatif de la courbe, et donc des paramètres habituellement pris en compte, en particulier:
PH (peak high): valeur maximale du signal.
TTP (time to peak): délai pour atteindre le maximum.
LT (lag time): délai de réaction
ETP (endogenous thrombine potentiel) ou AUC (area under curve : surface sous la courbe).

Tout écart de l'un de ces paramètres par rapport à la valeur de référence peut être interprété par le praticien pour détecter une anomalie du phénomène de coagulation.

Ainsi, lorsqu'on compare les courbes R₁ et FII, enregistrées dans les mêmes conditions et à la même température, on voit que la valeur de PH est fortement diminuée et que celle de TTP est considérablement augmentée, et même non déterminable sur le temps de mesure correspondant à la courbe. Cela peut être interprété comme une carence en prothrombine ou en un facteur déclenchant.

La figure 4 correspond à un graphique représenté à une échelle différente , en montrant le résultat d'une mesure d'un échantillon de plasma présentant une déficience en facteur V Leiden. Comme on peut le voir sur la figure 4. la valeur de PH est bien supérieure à la valeur PH de la référence. On observe en effet une valeur de TTP plus grande que celle de la référence (courbe R1) pour atteindre une valeur de PH à peine supérieure, ce qui traduit une activité atténuée du facteur VII

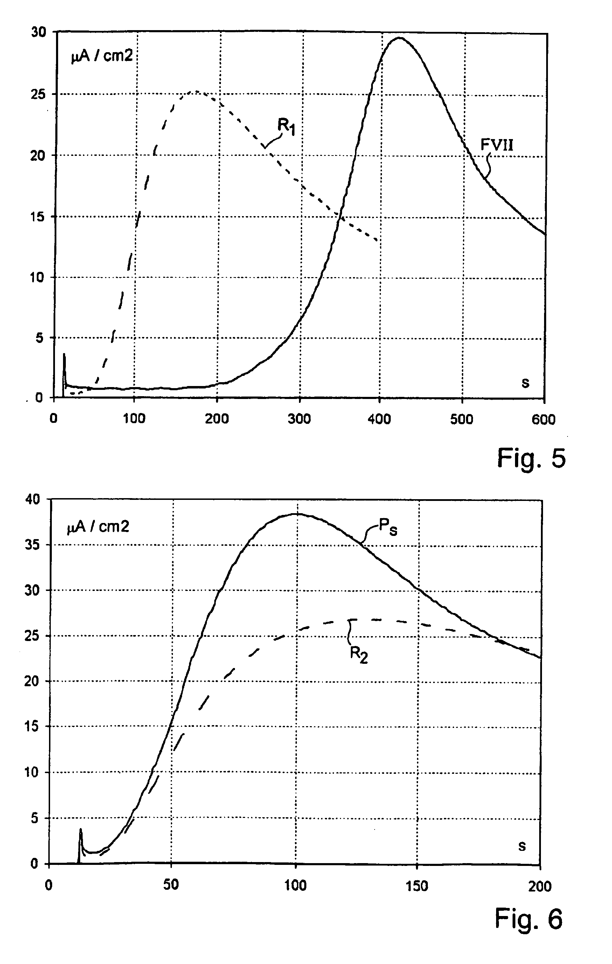

La figure 5 correspond, à la même échelle que la figure 3, à un graphique montrant une carence en facteur VII, à savoir la proconvertine, dont la présence contribue également à accroître la transformation de la prothrombine en thrombine. On observe en effet une valeur de TTP plus grande que celle de la référence (courbe R1) pour atteindre une valeur de PH à peine supérieure, ce qui traduit une activité atténuée du facteur VII

La figure 6 correspond, à la même échelle que la figure 4, à un graphique montrant une carence en protéine S dans le phénomène de coagulation. Comme on le voit, la valeur de TTP est peu modifiée par rapport à celle de la référence (courbe R2), et la valeur de PH correspondante présente une augmentation significative qui peut être interprétée comme une carence en protéine S.

En utilisant d'autres réactifs spécifiques appropriées il est possible de déterminer quels autres facteurs peuvent être responsables d'une anomalie du phénomène de coagulation, tels que le facteur VIII ou le facteur IX dont la carence correspond à une tendance hémophile, la protéine C, fantithrvmbine III, ou les lupusanticuagulants.

De même, sans sortir du cadre de l'invention le système peut être appliqué à d'autres fluides corporels en choisissant des substrats appropriés.

## Revendications

1. Système de détermination électrochimique de révolution de la concentration ou de l'activité d'au moins une enzyme protéolytique pour en détecter une éventuelle carence ou une activité anormale dans un faible échantillon d'un fluide corporel, le système comprenant :
- un assortiment de capteurs électrochimiques (10) pour la mesure d'une valeur représentative de l'évolution de la concentration ou de l'activité d'au moins une enzyme protéolytique d'un fluide corporel comprenant un support (11, 12) en forme de languette isolant au moins une électrode référence (15a) et une électrode de travail (14a), et comportant une zone de mesure (17) destinée à recevoir un échantillon de fluide corporel et une zone de connexion (16) desdites électrodes, un réactif étant immobilisé sur l'électrode de travail (14a) qui est spécifique d'une enzyme protéolytique donnée dont la composition incorpore au moins un substrat chimique dont un maillon terminal peut être coupé par ladite enzyme pour libérer des groupes chargés (LG), chaque capteur comportant un dispositif de repérage (19) spécifique permettant d'identifier pour lequel des facteurs de l'enzyme du fluide corporel le réactif est dédié ;
- un appareil de mesure (20) comportant autant de fentes de connexion (24) que de capteurs (10) dans ledit assortiment, chaque fente étant destinée à recevoir un desdits capteurs et dont le circuit électronique contrôle une source d'énergie permettant d'imposer entre les électrodes de chaque capteur un courant ou tension électrique, et mesure le signal électrique représentatif de la libération des groupes chargées (LG), et
- un appareil électronique (30) comportant un logiciel destiné à traiter le signal émis par l'appareil de mesure (20) afin d'afficher sur un écran de visualisation (32) une indication représentative de la libération des groupes chargés (LG) dans lesdits capteurs en fonction du temps
**caractérisé en ce que** le dispositif de repérage (19) comporte un bossage positionné sur la face supérieure du support (11) du capteur (10) en fonction du réactif utilisé et destiné à coopérer avec une encoche (29) agencée au-dessus d'une des fentes de connexion (24) de l'appareil de mesure (20) afin d'imposer l'introduction d'un seul type capteur (10) pour chaque fente (24).

2. Système selon la revendication 1, **caractérisé en ce que** le dispositif de repérage (19) est monté à une extrémité dudit capteur.

3. Système selon la revendication 1 ou 2, **caractérisé en ce que** la zone de mesure (17) comporte un capot de protection (18) formant un canal (18a) permettant d'amener le fluide corporel par capillarité jusqu'à la zone de mesure (17).

4. Système selon rune des revendications précédentes, **caractérisé en ce que** le volume de l'échantillon de fluide corporel prélevé est inférieur à 10 µl.

5. Système selon l'une des revendications 1 à 4, **caractérisé en ce que** le circuit électronique de l'appareil de mesure (20) est agencé pour effectuer une détermination chrono-ampérométrique.

6. Système selon l'une des revendications 1 à 5, **caractérisé en ce que** le logiciel de l'appareil électronique (30) comporte un module de détection des capteurs insérés permettant d'afficher des courbes de façon séquentielle ou en mosaïque.

7. Système selon l'une des revendications 1 à 6, **caractérisé en ce que** l'appareil de mesure (20) comporte en outre un dispositif de fermeture (23) permettant d'isoler du milieu extérieur les capteurs introduits (10) dans ledit appareil, et un dispositif de thermorégulation permettant de les maintenir à une température constante déterminée pendant toute la durée de la mesure.

8. Système selon l'une des revendications 1 à 6, **caractérisé en ce que** l'appareil de mesure (20) comporte en outre une sonde thermique destinée à mesurer la température ambiante afin que le logiciel de l'appareil électronique (30) sélectionne la courbe de référence en fonction de ladite température ambiante.

9. Système selon l'une des revendications 1 à 8, **caractérisé en ce que** l'appareil de mesure (20) comporte en outre un écran secondaire d'affichage (25) permettant d'afficher un paramètre global ou instantané de la mesure en cours.

10. Système selon l'une des revendications 1 à 9, **caractérisé en ce que** l'appareil de mesure (20) et l'appareil électronique (30) sont réunis dans une unique unité fonctionnelle.

11. Système selon l'une des revendications 1 à 10, **caractérisé en ce que** le fluide biologique est du sang, notamment du sang entier, dans lequel on détermine l'évolution de la concentration des fadeurs de coagulation, le temps de prothrombine (PT), le temps de thromboplastine partiellement activée (APPT) ou au contraire des inhibiteurs de coagulation pour en détecter une éventuelle carence, ou au contraire un excès, ou une activité anormale.

12. Système selon la revendication 11, **caractérisé en ce que** la composition du réactif spécifique comprend au moins un substrat oligopéptidique, dont un maillon terminal peut être séparé par un facteur de coagulation pour donner un groupe chargé (LG), une thromboplastine et un milieu tampon.

## Claims

1. System for the electrochemical determination of the evolution of the concentration or activity of at least one proteolytic enzyme for detecting a deficiency or abnormal activity thereof in a small sample of bodily fluid, the system including:
- an assortment of electrochemical sensors (10) measuring a representative signal of evolution of the concentration or activity of at least one proteolytic enzyme of bodily fluid including a support (11, 12) in the shape of a tongue insulating at least one reference electrode (15a) and one working electrode (14a), and comprising a measuring area (17) intended to receive said bodily fluid and a connecting area (16) of said electrode, a reagent for a given proteolytic enzyme being immobilised onto working electrode (14a), the composition of which includes at least one chemical substrate, an end link of which can be cut by said enzyme to release leaving groups (LG), each sensor including a marking device (19) corresponding to the determination of a specific enzyme;
- a measuring apparatus (20) including as many connecting slots (24) as there are sensors (10) of said assortment, each slot being intended to receive one of said sensors and whose electronic circuit, powered by an energy source, imposes, between the electrodes of each sensor, an electric current or voltage, and for receiving in return an electric signal representative of the release of the leaving groups (LG), and
- an electronic apparatus (30) including software for processing the signal transmitted by the measuring apparatus (20) to display on a display screen (32) an indication representative of the release of the leaving groups (LG) as a function of time
**characterized in that** the marking device (19) includes a raised portion located onto the support (11) upper face of the sensor (10), being function of incorporated reagent and intends to cooperate with a notch (29) arranged on one of upper side connecting slots (24) of the measuring apparatus (20) in order to compel introduction of a single kind of sensor (10) for each slot (24).

2. System according to claim 1, wherein said marking device (19) is located at one end of the sensor.

3. System according to claim 1 or 2, **characterized in that** the measuring area (17) includes a protective cap (18) forming a channel (18a) permitting to capillary bring the bodily fluid to the measuring area (17).

4. System according to any of the preceding claims, **characterized in that** the volume of the sample of bodily fluid taken is less than 10 µl.

5. System according to any of claims 1 to 4, **characterized in that** the electronic circuit of the measuring apparatus (20) is arranged for carrying out a chrono-amperometric determination.

6. System according to any of claims 1 to 5, **characterized in that** the electronic apparatus (30) software can differentiate the sensors to display curves sequentially or in a mosaic.

7. System according to any of claims 1 to 6, **characterized in that** the measuring apparatus (20) further includes a closing device (23) for insulating the sensors (10) inserted into said apparatus from the external medium, and a thermoregulation device for keeping the sensors at a determined constant temperature during the entire measurement.

8. System according to any of claims 1 to 6, **characterized in that** the measuring apparatus (20) further includes a thermal probe for measuring the ambient temperature and **in that** the electronic apparatus (30) software enables the reference curve to be selected as a function of said ambient temperature.

9. System according to any of claims 1 to 8, **characterized in that** the measuring apparatus (20) further includes a secondary display screen (25) for displaying an overall or instantaneous parameter of the measurement being carried out.

10. System according to any of claims 1 to 9, **characterized in that** the measuring apparatus (20) and the electronic apparatus (30) are united in a single operating unit.

11. System according to any of claims 1 to 10, **characterized in that** the biological fluid is blood, particularly whole blood, in which the evolution of the concentration of coagulation factors, the prothrombin time (PT), the partially activated thromboplastin time (APPT) or conversely coagulation inhibitors is determined to detect any deficiency, or conversely an excess, or abnormal activity.

12. System according to claim 11, **characterized in that** the composition of the specific reagent includes at least one oligopeptide substrate, an end link of which can be separated by a coagulation factor to give a leaving group (LG), a thromboplastin and a buffer medium.

## Patentansprüche

1. System zur elektrochemischen Bestimmung der Entwicklung der Konzentration oder der Aktivität wenigstens eines proteolytischen Enzyms, um daraus eine eventuelle Karenz oder eine anomale Aktivität zu detektieren, in einer kleinen Probe eines Körperfluids, wobei das System umfasst:
- eine Anordnung elektrochemischer Sensoren (10) für die Messung eines Wertes, der die Entwicklung der Konzentration oder die Aktivität wenigstens eines proteolytischen Enzyms eines Körperfluids repräsentiert, die einen Träger (11, 12) in Form einer Zunge, die wenigstens eine Referenzelektrode (15a) und eine Arbeitselektrode (14a) isoliert, enthält und eine Messzone (17) umfasst, die dazu vorgesehen ist, eine Körperfluidprobe aufzunehmen, und eine Verbindungszone (16) der Elektroden aufweist, wobei ein Reagenz auf der Arbeitselektrode (14a), das für ein gegebenes proteolytisches Enzym spezifisch ist, dessen Zusammensetzung wenigstens ein chemisches Substrat enthält, dessen Endglied durch das Enzym abgetrennt werden kann, um die geladenen Gruppen (LG) freizugeben, immobilisiert ist, wobei jeder Sensor eine spezifische Kennzeichnungsvorrichtung (19) enthält, die die Identifizierung ermöglicht, für welchen der Faktoren des Enzyms des Körperfluids das Reagenz bestimmt ist;
- eine Messvorrichtung (20), die ebensoviele Verbindungsschlitze (24) wie Sensoren (10) in der Anordnung aufweist, wobei jeder Schlitz dazu vorgesehen ist, einen der Sensoren aufzunehmen, und wovon die elektronische Schaltung eine Energiequelle steuert, die ermöglicht, zwischen die Elektroden jedes Sensors einen elektrischen Strom einzuleiten oder dazwischen eine elektrische Spannung anzulegen, und das elektrische Signal misst, das die Freisetzung der geladenen Gruppen (LG) repräsentiert, und
- eine elektronische Vorrichtung (30), die Software enthält, die dazu vorgesehen ist, das von der Messvorrichtung (20) ausgesendete Signal zu verarbeiten, um auf einem Anzeigeschirm (32) eine Angabe anzuzeigen, die die Freisetzung der geladenen Gruppen (LG) in den Sensoren in Abhängigkeit von der Zeit repräsentiert,
**dadurch gekennzeichnet, dass** die Kennzeichnungsvorrichtung (19) eine Erhöhung aufweist, die auf der oberen Fläche des Trägers (11) des Sensors (10) als Funktion des verwendeten Reagenz positioniert ist und dazu vorgesehen ist, mit einer Vertiefung (29) zusammenzuwirken, die über einem der Verbindungsschlitze (24) der Messvorrichtung (20) angeordnet ist, um die Einführung eines einzigen Sensortyps (10) für jeden Schlitz (24) zu erzwingen.

2. System nach Anspruch 1, **dadurch gekennzeichnet, dass** die Kennzeichnungsvorrichtung (19) an einem Ende des Sensors montiert ist.

3. System nach Anspruch 1 oder 2, **dadurch gekennzeichnet, dass** die Messzone (17) eine Schutzkappe (18) aufweist, die einen Kanal (18a) bildet, der ermöglicht, das Körperfluid durch Kapillarität bis zu der Messzone (17) zu führen.

4. System nach einem der vorhergehenden Ansprüche, **dadurch gekennzeichnet, dass** das Volumen der entnommenen Körperfluidprobe kleiner als 10 µl ist.

5. System nach einem der Ansprüche 1 bis 4, **dadurch gekennzeichnet, dass** die elektronische Schaltung der Messvorrichtung (20) dazu ausgelegt ist, eine chrono-ampèrometrische Bestimmung auszuführen.

6. System nach einem der Ansprüche 1 bis 5, **dadurch gekennzeichnet, dass** die Software der elektronischen Vorrichtung (30) ein Modul für die Detektion der eingesetzten Sensoren enthält, das ermöglicht, Kurven sequentiell oder mosaikartig anzuzeigen.

7. System nach einem der Ansprüche 1 bis 6, **dadurch gekennzeichnet, dass** die Messvorrichtung (20) außerdem eine Verschlussvorrichtung (23) enthält, die ermöglicht, die in die Vorrichtung eingeführten Sensoren (10) gegenüber dem äußeren Milieu zu isolieren, und eine Thermoregulierungsvorrichtung enthält, die ermöglicht, sie während der Dauer der Messung auf einer bestimmten konstanten Temperatur zu halten.

8. System nach einem der Ansprüche 1 bis 6, **dadurch gekennzeichnet, dass** die Messvorrichtung (20) außerdem eine Wärmesonde enthält, die dazu bestimmt ist, die Umgebungstemperatur zu messen, damit die Software der elektronischen Vorrichtung (30) die Referenzkurve als Funktion der Umgebungstemperatur auswählt.

9. System nach einem der Ansprüche 1 bis 8, **dadurch gekennzeichnet, dass** die Messvorrichtung (20) außerdem einen sekundären Anzeigeschirm (25) aufweist, der ermöglicht, einen globalen oder momentanen Parameter der laufenden Messung anzuzeigen.

10. System nach einem der Ansprüche 1 bis 9, **dadurch gekennzeichnet, dass** die Messvorrichtung (20) und die elektronische Vorrichtung (30) in einer einzigen funktionalen Einheit vereinigt sind.

11. System nach einem der Ansprüche 1 bis 10, **dadurch gekennzeichnet, dass** das biologische Fluid Blut, insbesondere Vollblut, ist, in dem die Entwicklung der Konzentration der Koagulationsfaktoren, die Prothrombin-Zeit (PT), die Zeit des teilweise aktivierten Thromboplastins (APPT) oder im Gegensatz dazu der Koagulationsverhinderer bestimmt wird, um daraus eine eventuelle Karenz oder im Gegensatz dazu einen Überschuss oder eine anomale Aktivität zu detektieren.

12. System nach Anspruch 11, **dadurch gekennzeichnet, dass** die Zusammensetzung des spezifischen Reagenz wenigstens ein Oligopeptid-Substrat, dessen Endglied durch einen Koagulationsfaktor abgetrennt werden kann, um eine geladene Gruppe (LG) zu ergeben, ein Thromboplastin und ein Puffermilieu enthält.
